# EUROPEAN PATENT SPECIFICATION

(11) **EP 0 856 530 B1**
(45) Date of publication and mention of the grant of the patent: **21.05.2003**
(21) Application number: 98105784.7
(22) Date of filing: 02.08.1995
(51) Int. Cl.: C08F 10/00, C08F 4/622, C08F 4/54

(54) **Polymerisation catalyst**
Polymerisationskatalysator
Catalyseur de polymérisation

(30) Priority: 02.08.1994 US 284797
(43) Date of publication of application: 05.08.1998
(62) Divisional of application: 95928725.1
(73) Proprietor: UNION CARBIDE CHEMICALS & PLASTICS TECHNOLOGY CORPORATION, Danbury, Connecticut 06817-0001 (US)
(72) Inventor: Cann, Kevin Joseph, Rocky Hill, New Jersey 08522 (US); Apecetche, Marie Angelica, Piscataway, New Jersey 08854 (US); Moorhouse, John Henry, Kendall Park, New Jersey 08824 (US); Muruganadam, Natarajan, Belle Meade, New Jersey 08502 (US); Smith, Gregory George, Belle Mead, New Jersey 08502 (US); Williams, Gary Harry, Flemington, New Jersey 08822 (US)
(74) Representative: Allard, Susan Joyce

(56) References cited:
- EP-A- 0 011 184
- EP-A- 0 599 096
- EP-A- 0 647 657
- WO-A-92/17510
- US-A- 4 575 538
- PATENT ABSTRACTS OF JAPAN vol. 007, no. 147 (C-173), 28 June 1983 & JP 58 061107 A (NIPPON GOSEI GOMU KK), 12 April 1983, -& DATABASE WPI Week 8320 Derwent Publications Ltd., London, GB; AN 83-48192k XP002070776 , 4 December 1983
- IWAKAZU HATTORI ET AL: "CHEMICAL MODIFICATION OF NEODYMIUM HIGH CIS-1,4 POLYBUTADIENE WITH STYRENEOXIDE" POLYMERS FOR ADVANCED TECHNOLOGIES, vol. 4, no. 7, 1 August 1993, pages 450-456, XP000396650

## Description

This invention relates to a polymerisation catalyst, particularly a catalyst which is suitable for producing polydienes such as polybutadiene and polyisoprene in a gas phase reactor such as a gas phase fluidized bed reactor.

Polydienes such as polybutadiene and polyisoprene have been manufactured for many. years by solution polymerization and more recently by mass or bulk polymerization processes. Various catalytic solution and bulk or mass processes for the polymerization of butadiene are known in the art to be suitable for producing polybutadiene with a high content of 1,4-cis units, which is particularly suited for the manufacture of tires, belting, and other molded or extruded rubber or elastomer articles.

In solution polymerisation butadiene is polymerized in an inert solvent or diluent which does not enter into the structure of or adversely affect, the resulting polymer. Such solvents are usually aliphatic, aromatic and cycloaliphatic hydrocarbons such as pentane, hexane, heptane; benzene, toluene, cyclohexane and the like. In bulk polymerizations, the reaction medium is essentially solventless, and the monomer is employed as a diluent.

The discovery of gas-phase fluidized bed and stirred reactor processes for the production of polymers, especially polyolefin polymers, made it possible to produce a wide variety of new polymers with highly desirable and improved properties. These gas-phase processes, especially the gas fluidized bed process for producing such polymers provided a means for producing polymers with a drastic reduction in capital investment expense and dramatic savings in energy usage as compared to other then conventional polymerization processes.

In a conventional gas fluidized bed process a gaseous stream containing one or more monomers is passed into a fluidized bed reactor containing a bed of growing polymer particles in a polymerization zone, while continuously or intermittently introducing a polymerization catalyst into the polymerization zone. The desired polymer product is withdrawn from the polymerization zone, degassed, stabilized and packaged for shipment, all by well known techniques. Because the polymerization reaction is exothermic, substantial heat is generated in the polymerization zone which must be removed to prevent the polymer particles from overheating and fusing together. This is accomplished by continuously removing unreacted hot gases from the polymerization zone and replacing them with cooler gases. The hot gases removed from the polymerization zone are compressed, cooled in a heat exchanger, supplemented by additional amounts of monomer to replace monomer polymerized and removed from the reaction zone and then recycled into the bottom of the reactor. Cooling of the recycled gases is accomplished in one or more heat exchanger stages. The sequence of compression and cooling is a matter of design choice but it is usually preferable to provide for compression of the hot gases prior to cooling. The rate of gas flow into and through the reactor is maintained at a level such that the bed of polymer particles is maintained in a fluidized condition. The production of polymer in a stirred bed reactor is very similar, differing primarily in the use of mechanical stirring means to assist in maintaining the polymer bed in a fluidized condition.

Conventional gas phase fluidized bed resin production is very well known in the art as shown, for example, by the disclosure appearing in United States Patent Nos. 4,379,758; 4,383,095 and 4,876,320, which are incorporated herein by reference.

The production of polymeric substances in gas phase stirred reactors is also well known in the art as exemplified by the process and equipment descriptions appearing in United States Patent No. 3,256,263.

More recently, in U.S. Patent Nos. 4,994,534 and 5,304588, it has been taught that sticky polymers, including polybutadiene rubbers, can be produced in a fluidized bed reactor in the presence of a catalyst in a polymerization reaction above the softening temperatures of the sticky polymers in the presence of an inert particulate material. The sticky polymers produced in the gas phase process are granular having a mixture of rubber and inert material with a core containing a majority of rubber while the shell contains a majority of inert material. Further, U.S. Patent No. 5,317,036 discloses gas phase polymerization processes which utilize unsupported, soluble catalysts, such as, for example, transition metal coordination catalysts. The catalysts are introduced into the reactor, such as a fluidized bed, as a solution. The polyolefins produced by the process may contain dienes. EP 0 647 657 A1 discloses supported rare earth catalysts for gas phase polymerization of conjugated dienes.

For many years it was erroneously believed that to allow liquid of any kind to enter into the polymerization region of a gas phase reactor would inevitably lead to agglomeration of resin particles, formation of large polymer chunks and ultimately complete reactor shut-down. This concern caused gas phase polymer producers to carefully avoid cooling the recycle gas stream entering the reactor to a temperature below the condensation temperature of any of the monomers employed in the polymerization reaction.

Comonomers such as hexene-1, 4-methyl-pentene, and octene-1, are particularly valuable for producing ethylene copolymers. These higher alpha olefins have relatively high condensation temperatures. Due to the apprehension that liquid monomers in the polymerization zone would lead to agglomeration, chunking and ultimately shut down the reactor, production rates, which depend upon the rate at which heat is removed from the polymerization zone, were severely constrained by the perceived need to maintain the temperature of the cycle gas stream entering the reactor at temperature safely above the condensation temperature of the highest boiling monomer present in the cycle gas stream.

Even in the case of polymerization reactions conducted in stirred reactors, care was exercised to maintain the resin bed temperature above the condensation temperature of the recycle gas stream components.

To maximize heat removal it was not unusual to spray or inject liquid into or onto the polymer bed where it would immediately flash into a gaseous state by exposure to the hotter recycle gas stream. A limited amount of additional cooling was achieved by this technique by the Joules-Thompson effect but without ever cooling the recycle gas stream to a level where condensation might occur. This approach typically involved the laborious and energy wasting approach of separately cooling a portion of the cycle gas stream to obtain liquid monomer for storage and subsequent separate introduction into or onto the polymerization bed. Examples of this procedure are found in United States Patent Nos. 3,254,070; 3,300,457; 3,652,527 and 4,012,573.

It was discovered later, contrary to the long held belief that the presence of liquid in the cycle gas stream would lead to agglomeration and reactor shut-down, that it is indeed possible to cool the entire cycle gas stream to a temperature where condensation of significant amounts of monomer would occur without the expected dire results when these liquids were introduced into the reactor in temperature equilibrium with the recycle gas stream. Cooling the entire cycle gas stream produces a two-phase gas-liquid mixture in temperature equilibrium with each other so that the liquid contained in the gas stream does not immediately flash into vapor. Instead a substantially greater amount of cooling takes place because the total mass of both gas and liquid enters the polymerization zone at a substantially lower temperature than previously thought possible. This process led to substantial improvements in the yield of polymers produced in the gas phase, especially where comonomers which condense at relatively low temperatures are used. This procedure, commonly referred to as "condensing mode" operation, is described in detail in United States Patent Nos. 4,543,399 and 4,588,790. In condensing mode operation the two-phase gas-liquid mixture entering the polymerization zone is heated quite rapidly and is completely vaporized within very short distance after entry into the polymerization zone. Even in the largest commercial reactors, all liquid has been vaporized and the temperature of the then totally gaseous cycle gas stream raised substantially by the exothermic nature of the polymerization reaction soon after entry into the polymerization zone. The ability to operate a gas phase reactor in condensing mode was believed possible due to the rapid heating of the two-phase gas liquid stream entering the reactor coupled with efficient constant back mixing of the fluidized bed leaving no liquid present in the polymer bed more than a short distance above the entry level of the two-phase gas-liquid recycle stream.

We have now found that liquid monomer may be present throughout the entire polymer bed provided that the liquid monomer present in the bed is adsorbed on or absorbed in solid particulate matter present in the bed, such as the polymer being produced or fluidization aids present in the bed, so long as there is no substantial amount of free liquid monomer. This discovery makes it possible to produce polymers in a gas phase reactor with the use of monomers having condensation temperatures much higher than the temperatures at which conventional polyolefins are produced in gas phase reactors. Another way of viewing this discovery is that it is now possible to produce polymers using readily condensable monomers (e.g., 1,3-butadiene, having a normal boiling point of -4.5°C) in a gas phase reactor under conditions at which the monomer would be expected to be present as a liquid. Furthermore, it had been previously believed that gas phase processes for producing polymers with some or all of the monomers having low to moderate condensation temperatures were impractical because the amount of polymer produced per catalyst particle was too low at all monomer concentrations that had condensation temperatures below the temperature in the polymerization zone. This discovery now makes it economically practical to produce polymer with monomers at concentrations where they have condensation temperatures higher than the temperature in the polymerization zone, such that liquid monomer is present throughout the entire polymer bed provided that the liquid monomer present in the bed is adsorbed on or absorbed in solid particulate matter, the polymer bed, and/or the forming polymer product present in the polymerization zone of the reactor. This above discovery makes possible the gas phase production of classes of polymers which previously were thought not capable of production in a continuous gas phase process.

Another benefit of the discovery is that operation with monomer present as liquid dissolved in the polymer gives a greater concentration of monomer at the active catalyst site than operation with monomer not dissolved, i.e., present only in the gas phase. This should maximize the productivity of the catalyst for making polymer. Still another benefit of the discovery is that heat transfer within the polymer particles should be improved due to removal of heat by monomer evaporation. This should lead to more uniform polymer particle temperatures, more uniform polymer properties, less polymer fouling, and possibly improved polymer morphology than operation with monomer not dissolved, i.e., present only in the gas phase.

The present invention provides a neodymium polymerisation catalyst particularly, but not exclusively, for use in the above processes. The present invention provides a polymerisation catalyst comprising:
(A) rare earth metal component selected from the group consisting of (1) a reaction mixture obtainable by treating a silica support, a carbon black support or a mixed support thereof with a mono- or di-ethyl aluminium chloride, adding a neodymium carboxylate in the presence of a solvent to form a slurry, and removing the solvent or (2) a reaction mixture obtainable by dissolving a neodymium carboxylate in a first solvent, adding silica or carbon black, and removing the solvent to obtain a dry solid, contacting the dry solid with a solution containing a mono- or di-ethyl aluminium chloride in a second solvent, and removing the second solvent; and
B) a co-catalyst selected from the group consisting of a diisobu aluminium hydride, triisobutyl aluminium, and a mixture thereof.

The catalyst of the present invention can be used in a process for producing polybutadiene or polyisoprene in a stirred bed or gas fluidized polymerization vessel having a polymerization zone under polymerization reaction conditions, which process comprises:
(i) introducing butadiene or isoprene monomer into said polymerization zone containing a bed of growing polymer particles in the presence of an inert particulate material and optionally at least one inert gas;
(ii) continuously or intermittently introducing a polymerization catalyst containing a rare earth metal component, a co-catalyst, and optionally a promoter into said polymerization zone;
(iii) continuously or intermittently withdrawing polybutadiene or polyisoprene product from said polymerization zone;
(iv) withdrawing unreacted butadiene or isoprene from said polymerization zone, compressing and cooling said butadiene or isoprene and said inert gas when present, while maintaining the termperature within said polymerization zone below the dew point of the monomer present in said polymerization zone.

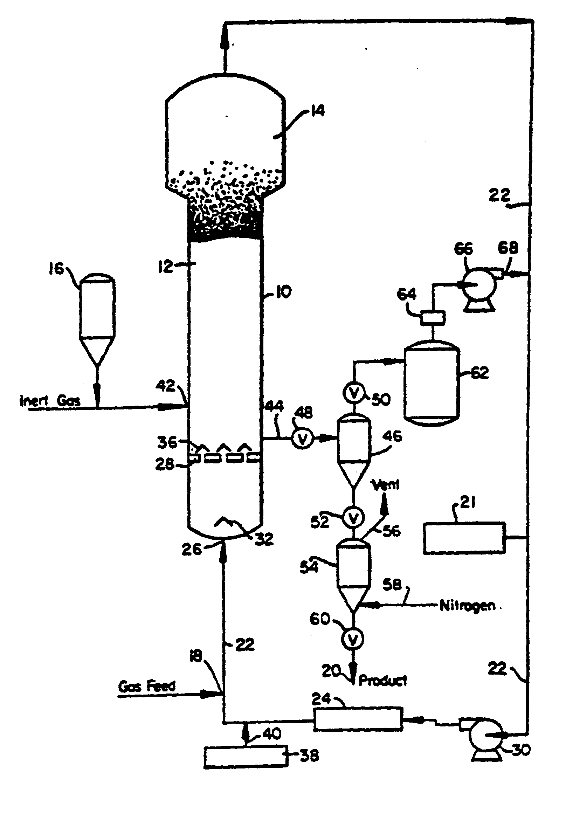

A fluidized bed reaction system which is particularly suited to the production of polybutadiene and polyisoprene is illustrated in the drawing.

While not limited to any particular type or kind of polymerization reaction, this invention is particularly well suited to use in olefin polymerization reactions involving homopolymerization and copolymerization of relatively high boiling or readily condensable monomers such as butadiene and isoprene.

Examples of higher boiling or readily condensable monomers capable of undergoing olefinic polymerization reactions using the catalyst of the present invention are the following:
A. higher molecular weight alpha olefins such as decene-1, dodecene-1, isobutylene, or styrene.
B. dienes such as hexadiene, vinyl cyclohexene, dicyclopentadiene, butadiene, isoprene or ethylidene norbornene.
C. polar vinyl monomers such as acrylonitrile, maleic acid esters, vinyl acetate, acrylate esters, methacrylate esters or vinyl trialkyl silanes.

These higher boiling or readily condensable monomers can be homopolymerized using the catalyst of the present invention with the use of an inert gas as the gaseous component of the two phase gas-liquid mixture cycled through the reactor. Suitable inert materials for this purpose include nitrogen, argon, and saturated hydrocarbons which remain gaseous at a temperature below the temperature selected to be maintained in the polymerization zone.

The higher boiling or readily condensable monomers can also be copolymerized with one or more lower boiling monomers such as ethylene, propylene and butene, as well as other higher boiling monomers such as those mentioned above, the only requirement being that there be a sufficient difference in the condensation temperatures of the higher boiling or readily condensable monomer and at least one lower boiling monomer or inert substance as will allow enough gas to be present in the cycle gas stream to permit practical, steady state, continuous operation.

The higher boiling or readily condensable monomers can be directly introduced into the polymerization zone or carried into the polymerization zone as with the recycle gas stream or a combination of both. The temperature within said polymerization zone is preferably maintained below the condensation temperature of the monomer (e.g., 1,3-butadiene or isoprene) present in said polymerization zone. The conditions (e.g., temperature, pressure, monomer(s) concentration) within said polymerization zone are preferably such that essentially no liquid is present in said polymerization zone that is not adsorbed on or absorbed in solid particulate matter. Alternatively, the conditions within said polymerization zone are maintained such that a portion of the monomer in the polymerization zone is a liquid that is not absorbed in the solid particulate matter.

The catalyst of the present invention employed in the polymerization zone is a rare earth metal catalyst. The rare earth metal catalysts have a metal component, which is neodymium carboxylate, a co-catalyst, and optionally a promoter. Preferably, a promoter is not employed in the catalyst of the polymerization process of this invention. In general, the rare earth metal component of the catalyst can be soluble or insoluble, or spray dried in either the presence or absence of a filler. Alternatively, the catalyst can be introduced to the polymerization zone in the form of a prepolymer using techniques known to those skilled in the art.

Support materials include carbon black or silica or mixtures of carbon black and silica. A typical silica support is a solid, particulate, porous material essentially inert to the polymerization. It is used as a dry powder having an average particle size of from 10 to 250 µm and preferably 30 to 100 µm; a surface area of at least 200 square meters per gram and preferably at least 250 square meters per gram; and a pore size of at least 10 nm (100 Angstroms) and preferably at least 20nm (200 Angstroms). Generally, the amount of support used is that which will provide 0.1 to 1.0 millimole of rare earth metal per gram of support. Two types of carbon black are preferably used as support. DARCO G-60 (pH of water extract = 5) is used as dry powder having a surface, area of 505 square meters per gram, average particle size of 100 µm, and porosity of 1.0 to 1.5 cubic centimeter per gram. NORIT A (pH of water extract = 9 - 11) used as a dry powder, has a surface area of 720 square meters per gram, average particle size of 45 to 80 µm.

Illustrative neodymium carboxylates can include neodymium octanoate, neodymium octoate, neodymium 2,2-diethylhexanoate, neodymium 2-ethylhexoate, neodymium 2-ethylcctoate and neodymium 2,2-diethyl heptanoate. Neodymium neodecanoate, octanoate, or versatate give particularly good results.

The catalyst modifiers and co-catalysts consist of aluminum alkyl halides and trialkyl aluminum compounds such as the following:

Alkylaluminum halides can be a compound having the formula AlR₍₃₋ₐ₎Xₐ wherein each R is independently alkyl having 1 to 14 carbon atoms; each X is independently chlorine, bromine, or iodine; and a is 1 or 2 or a mixture of compounds having the formulas AlR₍₃₋ₐ₎Xₐ and AlR₃ wherein R, X, and a are the same as above.

Specifically halogen containing modifiers are mono- or di-ethyl aluminium chlorides Diethylaluminum chloride (DEAC) is most preferred.

Co-catalysts that are employed with rare earth metal compounds in the present invention include triisobutylaluminum (TIBA), or a dialkyl aluminum hydride that is diisobutyl aluminium hydride (DIBAM) or a mixture of said dialkyl aluminum hydride and said trialkyl aluminum.

Promoters that can be used with neodymium carboxylates include Lewis acids such as BCl₃, AlCl₃, ethylaluminum dichloride, ethylaluminum sequichloride, diethylaluminum chloride, and other alkyl radical derivatives of these compounds. Organohalide derivatives of the formula 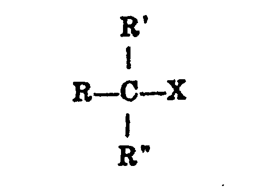 in which X is Cl or Br, R is H, alkyl, aryl, alkylaryl, chloro or bromo alkyl, alkoxy or epoxy; R' is alkyl, aryl, H, Cl or Br; R" is alkyl, aryl, chloro or bromo alkyl, chloro or bromo aryl, vinyl, Cl or Br; or R' + R" is oxygen, or saturated or unsaturated cycloalkyl. IF R=R'=H, then R" is only of aromatic nature. The alkyl radicals can be either saturated or unsaturated, linear or branched, and contain from 1 to 18 carbon atoms.

Typical examples of organohalide derivatives which can be used as catalytic components of the present invention are benzoyl, propionyl, benzyl, benzylidene or tertiary butyl chlorides or bromides, methyl chloroformate or bromoformate, chlorodiphenylmethane or chlorotriphenylmethane.

The catalyst can be prepared by treating the support material with the metal component, cocatalyst and optional promoter in any order in an inert solvent or diluent. In general the metal component can be impregnated on a support by well known means such as by dissolving the metal compound in a solvent or diluent such as a hydrocarbon or ether (including aliphatic, cycloaliphatic or aromatic compounds such as pentane, isopentane, hexane, cyclohexane, benzene, toluene, and tetrahydrofuran) in the presence of the support material and then removing the solvent or diluent by evaporation such as under reduced pressure. Alternatively, the rare earth metal component can be dissolved in a solvent or diluent such as a hydrocarbon or tetrahydrofuran and spray dried to generate a well-shaped catalyst precursor having little or no silica or other inorganic solids content, if desired.

A preferred method for making the catalyst of this invention involves impregnating a silica support, a carbon black support, or a mixed support of the two with a rare earth metal containing compound. The amount of metal impregnated on the support can range between 0.1 and 1.0 mole/g catalyst. An organic alkyl aluminum compound may be added prior, during or subsequent to the impregnation step, either in a hydrocarbon or oxygenated solvent such as THF. The catalyst may be isolated as a dry solid or used as a slurry in a diluent. The catalyst may also be prepared without a support by simple contact of the metal with the alkyl aluminum compound to form a solution or slurry which is fed directly to the reactor. The A1 to metal ratio in the catalyst preparation step may vary between 0.5 to 5.0. The polymerization metal may be used without aluminum treatment when an organohalide promoter or aluminum alkyl halide is also fed to the reactor with the cocatalyst. When MAO is used as the cocatalyst no halide source is required.

A preferred procedure for making the rare earth catalyst of the invention comprises the sequential steps of (A) treating a silica support, carbon black support, or mixture of the two support materials with a mono- or di- ethyl aluminum chloride or a mixture of the two chlorides in a hydrocarbon solvent thereby forming a slurry; (B) adding the rare earth compound (the neodymium compound); and (C) removing the hydrocarbon solvent or diluent. Catalysts which are preferred in the process of this invention are (I) a neodymium neodecanoate, neodymium octoate, or neodymium versatate as the metal component, and an organic alkyl aluminum compound such as diethyl aluminum chloride to form the catalyst in a diluent such as n-hexane or cyclohexane and (II) a neodymium carboxylate such as neodymium neodecanoate, neodymium octoate, or neodymium versatate as the metal component is impregnated on silica by dissolving the neodymium compound in THF, adding silica, followed by solvent removal. The dry solid is added to a hydrocarbon (e.g. hexane) solution containing an alkylaluminum chloride with subsequent removal of the hydrocarbon solvent. These catalysts are fed to the reactor with a co-coatalyst selected from the group consisting of diisobutyl aluminum hydride (DIBAH), triisobutylaluminum, or a mixture of diisobutyl aluminum hydride and triisobutylaluminum (TIBA). These catalysts are preferred because they have little or no induction period and remain catalytically active for a long period of time. The catalyst I above can be fed directly to the reactor. Still another catalyst can be prepared by forming a reaction mixture by (i) contacting a neodymium carboxylate, with a mono-ethyl aluminum, dichloride, a di-ethyl aluminum chloride or a mixture of the mono- and di-ethyl aluminum chloride (ii) depositing the mixture on a silica support in the presence of a solvent to form a slurry and (iii) removing said solvent; and adding a co-catalyst selected from the group consisting of (i) dialkyl aluminum hydride, (ii) a trialkyl aluminum, and (iii) a mixture of a dialkyl aluminum hydride and a trialkyl aluminum.

Fluidization aids employed in the invention can be inert particulate materials which are chemically inert to the reaction. Examples of such fluidization aids include carbon black, silica, clays and other like materials such as talc. Organic polymeric materials can also be employed as a fluidization aid. Carbon blacks and silicas are the preferred fluidization aids with carbon black being the most preferred. The carbon black materials employed have a primary particle size of from 10 to 100 nanometers and an average size of aggregate (primary structure) of from 0.1 to 10 µm. The specific surface area of the carbon black is from 30 to 1,500 m²/g and the carbon black displays a dibutylphthalate (DBP) absorption of from 80 to 350 cc/100 grams.

Silicas which can be employed are amorphous and have a primary particle size of from 5 to 50 nanometers and an average size of aggregate of from 0.1 to 10 µm. The average size of agglomerates of silica is from 2 to 120 µm. The silicas employed have a specific surface area of from 50 to 500 m²/g and a dibutylphthalate (BDP) absorption of from 100 to 400 cc/100 grams.

Clays which can be employed according to the invention have an average particle size of from 0.01 to 10 microns and a specific surface area of from 3 to 30 m2/gm. They exhibit oil absorption of from 20 to 100 gms per 100 gms.

Organic polymeric substances which can be used include polymers and copolymers of ethylene, propylene, butene, and other alpha olefins and polystyrene, in granular or powder form. These organic polymeric materials have an average particle size ranging from 0.01 to 100 microns, preferably 0.01 to 10 microns.

In general, the amount of fluidization aid utilized generally depends on the type of material utilized and the type of polybutadiene or polyisoprene produced. When utilizing carbon black or silica as the fluidization aid, they can be employed in amounts of from 0.3% to 80% by weight, preferably 5% to 60%, and most preferably 10% to 45%, based on the weight of the final product (polybutadiene or polysioprene) produced. When clays or talcs are employed as the fluidization aid, the amount can range from 0.3% to 80% based on the weight of the final product, preferably 12% to 75% by weight. Organic polymeric materials are used in amounts of from 0.1% to 50% by weight, preferably 0.1% to 10% based on the weight of the final polymer product produced.

The fluidization aid can be introduced into the reactor at or near the top of the reactor, at the bottom of the reactor, or to the recycle line directed into the bottom of the reactor. Preferably, the fluidization aid is introduced at or near the top of the reactor or above the fluidized bed. It is preferred to treat the fluidization aid prior to entry into the reactor to remove traces of moisture and oxygen. This can be accomplished by purging the material with nitrogen gas and heating by conventional procedures. The fluidization aid can be added separately or combined with one or more butadiene monomers, or with a soluble unsupported catalyst. Preferably, the fluidization aid is added separately.

A fluidized bed reaction system which is particularly suited to production of polymeric materials is illustrated in the drawing. With reference thereto, the reactor 10 consists of a reaction zone 12 and a velocity reduction zone 14.

In general, the height to diameter ratio of the reaction zone can vary in the range of 2.7:1 to 4.6:1. The range, of course, can vary to larger or smaller ratios and depends upon the desired production capacity. The cross-sectional area of the velocity reduction zone 14 is typically within the range of 2.6 to 2.8 multiplied by the cross-sectional area of the reaction zone 12.

The reaction zone 12 includes a bed of growing polymer particles, formed polymer particles and a minor amount of catalyst particles fluidized by the continuous flow of polymerizable and modifying gaseous components in the form of make-up feed and recycle fluid through the reaction zone. To maintain a viable fluidized bed, the superficial gas velocity through the bed must exceed the minimum flow required for fluidization, and preferably is at least 0.03 m/sec (0.1 ft/sec) above minimum flow. Ordinarily, the superficial gas velocity does not exceed 1.5 m/sec (5.0 ft/sec) and usually no more that 0.76 m/sec (2.5 ft./sec) is sufficient.

It is essential that the bed always contain particles to prevent the formation of localized "hot spots" and to entrap and distribute catalyst throughout the reaction zone. On start up, the reactor is usually charged with a bed of particulate polymer particles. Such particles may be identical in nature to the polymer to be formed or they may be different. When different, they are withdrawn with the desired formed polymer particles as the first product. Eventually, a fluidized bed of desired polymer particles supplants the start-up bed.

A partially or totally activated precursor composition and-or catalyst used in the fluidized bed is preferably stored for service in a reservoir 16 under a blanket of a gas which is inert to the stored material, such as nitrogen or argon.

Fluidization is achieved by a high rate of fluid recycle to and through the bed, typically on the order of 50 to 150 times the rate of feed of make-up fluid. The fluidized bed has the general appearance of a dense mass of individually moving particles as created by the percolation of gas through the bed. The pressure drop through the bed is equal to or slightly greater than the weight of the bed divided by the cross-sectional area. It is thus dependent on the geometry of the reactor.

Make-up fluid can be fed to the bed at point 18. The composition of the make-up stream is determined by a gas analyzer 21. The gas analyzer determines the composition of the recycle stream and the composition of the make-up stream is adjusted accordingly to maintain an essentially steady state gaseous composition within the reaction zone.

The gas analyzer is a conventional gas analyzer which operates in a conventional manner to determine the recycle stream composition to facilitate maintaining the ratios of feed stream components. Such equipment is commercially available from a wide variety of sources. The gas analyzer 21 is typically positioned to receive gas from a sampling point located between the velocity reduction zone 14 and heat exchanger 24.

The higher boiling or readily condensable monomers can be introduced into the polymerization zone in various ways including direct injection through a nozzle (not shown in the drawing) into the bed or by spraying onto the top of the bed through a nozzle (not shown) positioned above the bed, which may aid in eliminating some carryover of fines by the cycle gas stream. If the rate of monomer feed is relatively small, heavier monomers can be introduced into the polymerization zone simply by suspension in the cycle gas stream entering the bottom of the reactor.

To ensure complete fluidization, the recycle stream and, where desired, part of the make-up stream are returned through recycle line 22 to the reactor at point 26 below the bed. There is preferably a gas distributor plate 28 above the point of return to aid in fluidizing the bed. In passing through the bed, the recycle stream absorbs the heat of reaction generated by the polymerization reaction.

The portion of the fluidizing stream which has not reacted in the bed is removed from the polymerization zone, preferably by passing it into velocity reduction zone 14 above the bed where entrained particles can drop back into the bed.

The recycle stream is compressed in a compressor 30 and then passed through a heat exchange zone where heat is removed before it is returned to the bed. The heat exchange zone is typically a heat exchanger 24 which can be of the horizontal or vertical type. If desired, several heat exchangers can be employed to lower the temperature of the cycle gas stream in stages. It is also possible to locate the compressor downstream from the heat exchanger or at an intermediate point between several heat exchangers. After cooling, the recycle stream is returned to the reactor at its base 26 and to the fluidized bed through gas distributor plate 28. A gas deflector 32 can be installed at the inlet to the reactor to prevent contained polymer particles from settling out, agglomerating into a solid mass, and to prevent liquid accumulation at the bottom of the reactor, as well to facilitate easy transitions between processes which contain liquid in the cycle gas stream and those which do not and vice versa. Illustrative of gas deflectors suitable for this purpose is the apparatus described in U.S. Patent No. 4,933,149.

The selected temperature of the bed is maintained at an essentially constant temperature under steady state conditions by constantly removing the heat of reaction. No noticeable temperature gradient appears to exist within the upper portion of the bed. A temperature gradient can exist in the bottom of the bed in a layer of 15 to 30 cm (6 to 12 inches), between the temperature of the inlet fluid and the temperature of the remainder of the bed.

Good gas distribution plays an important role in the operation of the reactor. The fluidized bed contains growing and formed particulate polymer particles, as well as catalyst particles. As the polymer particles are hot and possibly active, they must be prevented from settling, for if a quiescent mass is allowed to exist, any active catalyst contained therein may continue to react and cause fusion. Diffusing recycle fluid through the bed at a rate sufficient to maintain fluidization throughout the bed is, therefore, important.

Gas distribution plate 28 is a preferred means for achieving good gas distribution and may be a screen, slotted plate, perforated plate, a plate of the bubble-cap type and the like. The elements of the plate may all be stationary, or the plate may be of the mobile type disclosed in U.S. Patent No. 3,298,792. Whatever its design, it must diffuse the recycle fluid through the particles at the base of the bed to keep the bed in a fluidized condition, and also serve to support a quiescent bed of resin particles when the reactor is not in operation.

The preferred type of gas distributor plate 28 is metal and has holes distributed across its surface. The holes are normally of a diameter of about 1.25 cm (about 1/2 inch). The holes extend through the plate. Over each hole there is positioned a triangular angle iron identified as 36 which is mounted on plate 28. The angle irons serve to distribute the flow of fluid along the surface of the plate so as to avoid stagnant zones of solids. In addition they prevent the polymer from flowing through the holes when the bed is settled.

Any fluid inert to the catalyst and reactants can also be present in the recycle stream. An activator compound, if utilized, is preferably added to the reaction system downstream from heat exchanger 24, in which case the activator may be fed into the recycle system from dispenser 38 through line 40.

In the practice of this invention operating temperatures can extend over a range of from -100°C to 150°C with temperatures ranging from 20°C to 120°C being preferred.

The fluid-bed reactor can be operated at pressures up to 6890 kPa (1000 psi) and preferably at a pressure of from 689 to 4134 kPa (100 psi to 600 psi). Operation at higher pressures favors heat transfer as an increase in pressure increases the unit volume heat capacity of the gas.

The partially or totally activated precursor composition (e.g., neodymium with an alkyl halide) and/or catalyst (hereinafter collectively referred to as catalyst) is injected into the bed at a point 42 which is above distributor plate 28. Preferably, the catalyst is injected at a point in the bed where good mixing with polymer particles occurs. Injecting the catalyst at a point above the distribution plate provides for satisfactory operation of a fluidized bed polymerization reactor. Injection directly into the fluidized bed aids in distributing the catalyst uniformly throughout the bed and tends to avoid the formation of localized spots of high catalyst concentration which can cause "hot spots" to form. Injection of the catalyst into the reactor above the bed can result in excessive catalyst carryover into the recycle line where polymerization can occur leading to plugging of the line and heat exchanger.

A supported catalyst can be injected into the reactor by various techniques. It is preferred, however, to continuously feed the catalyst into the reactor utilizing a catalyst feeder as disclosed, e.g., in U.S. Patent No. 3,779,712. The catalyst is preferably fed into the reactor at a point 20 to 40 percent of the reactor diameter away from the reactor wall and at a height of 5 to 30 percent of the height of the bed.

A gas which is inert to the catalyst, such as nitrogen or argon, is preferably used to carry the catalyst into the bed.

The rate of polymer production in the bed depends on the rate of catalyst injection and the concentration of monomer(s) in the reactor. The production rate is conveniently controlled by simply adjusting the rate of catalyst injection.

Since any change in the rate of catalyst injection will change the reaction rate and thus the rate at which heat is generated in the bed, the temperature of the recycle stream entering the reactor is adjusted upwards and downwards to accommodate any change in the rate of heat generation. This ensures the maintenance of an essentially constant temperature in the bed. Complete instrumentation of both the fluidized bed and the recycle stream cooling system is, of course, useful to detect any temperature change in the bed so as to enable either the operator or a conventional automatic control system to make a suitable adjustment in the temperature of the recycle stream.

Under a given set of operating conditions, the fluidized bed is maintained at essentially a constant height by withdrawing a portion of the bed as product at the rate of formation of the particulate polymer product. Since the rate of heat generation is directly related to the rate of product formation, a measurement of the temperature rise of the fluid across the reactor (the difference between inlet fluid temperature and exit fluid temperature) is indicative of the rate of particular polymer formation at a constant fluid velocity if no or negligible vaporizable liquid is present in the inlet fluid.

On discharge of particulate polymer product from reactor 10, it is desirable and preferable to separate fluid from the product and to return the fluid to the recycle line 22. There are numerous ways known to the art to accomplish this. One preferred system is shown in the drawings. Thus, fluid and product leave reactor 10 at point 44 and enter product discharge tank 46 through valve 48, which may be a ball valve which is designed to have minimum restriction to flow when opened. Positioned above and below product discharge tank 46 are conventional valves 50, 52 with the latter being adapted to provide passage of product into product surge tank 54. Product surge tank 54 has venting means illustrated by line 56 and gas entry means illustrated by line 58. Also positioned at the base of product surge tank 54, is a discharge valve 60 which when in the open position discharges product for conveying to storage. Valve 50 when in the open position releases fluid to surge tank 62. Fluid from surge tank 62 is directed through a filter absorber 64 and thence through a compressor 66 and into recycle line 22 through line 68.

In a typical mode of operation, valve 48 is open and valves 50, 52 are in a closed position. Product and fluid enter product discharge tank 46. Valve 48 closes and the product is allowed to settle in product discharge tank 46. Valve 50 is then opened permitting fluid to flow from product discharge tank 46 to surge tank 62 from which it is continually compressed back into recycle line 22. Valve 50 is then closed and valve 52 is opened and any product in product discharge tank 46 flows into product surge tank 54. Valve 52 is then closed. The product is purged with inert gas, preferably nitrogen, which enters product surge tank 54 through line 58 and is vented through line 56. Product is then discharged from product surge tank 54 through valve 60 and conveyed through line 20 to storage.

The particular timing sequence of the valves is accomplished by the use of conventional programmable controllers which are well known in the art. Moreover, the valves can be kept substantially free of agglomerated particles by directing a stream of gas periodically through the valves and back to the reactor.

Another preferred product discharge system which may be alternatively employed is that disclosed and claimed in the copending U.S. patent application of Robert G. Aronson filed July 28, 1981, Ser. No 287,815 and entitled "Fluidized Bed Discharge System" (now U.S. Patent No. 4,621,952). Such a system employs at least one (parallel) pair of tanks comprising a settling tank and a transfer tank arranged in series and having the separated gas phase returned from the top of the settling tank to a point in the reactor near the top of the fluidized bed. Such alternative preferred product discharge system obviates the need for a recompression, lines 64, 66, 68, as shown in the system of the drawing.

The fluidized-bed reactor is equipped with an adequate venting system (not shown) to allow venting the bed during start up and shut down. The reactor does not require the use of stirring and/or wall scraping. The recycle line 22 and the elements therein (compressor 30, heat exchanger 24) should be smooth surfaced and devoid of unnecessary obstructions so as not to impede the flow of' recycle fluid or entrained particles.

Illustrative of the polymers which can be produced using a catalyst of the present invention are the following:
Polyisoprene
Polystyrene
Polybutadiene
SBR (polymer of butadiene copolymerized with styrene)
ABS (polymer of acrylonitrile, butadiene and styrene)
Nitrile (polymer of butadiene copolymerized with acrylonitrile)
Butyl (polymer of isobutylene copolymerized with isoprene)
EPR (polymer of ethylene copolymerized with propylene)
EPDM (polymer of ethylene copolymerized with propylene and a diene such as hexadiene, dicyclopentadiene, or ethylidene norbornene)
Neoprene (polychloroprene)
Silicone (polydimethyl siloxane)
Copolymer of ethylene and vinyltrimethoxy silane
Copolymer of ethylene and one or more of acryonitrile, maleic acid esters, vinyl acetate, acrylic and methacrylic acid esters and the like.

When it is desired to produce polymers or copolymers using one or more monomers which are all relatively high boiling or readily condensable and which form liquids under the temperature and pressure conditions which are preferred for gas phase fluidized bed production in accordance with the invention, it is preferable to employ an inert substance which will remain gaseous under the conditions selected for polymerization in the fluidized bed. Suitable for this purpose are inert gases such as nitrogen, argon, neon or krypton. Also useful are saturated hydrocarbons such as ethane, propane or butane as well as halogen substituted alkanes such as freon. Other materials which remain gaseous under the desired conditions, such as carbon dioxide, provided they are essentially inert and do not affect catalyst performance, can also be employed.

Nitrogen, because of its physical properties and relatively low cost is a preferred medium for the manufacture of polymers from higher boiling or readily condensable monomers such as styrene, vinyl acetic acid, acrylonitrile, methylacrylate or methylmethacrylate. Alkanes such as ethane and propane which remain gaseous at relatively low temperatures are also preferred.

Conventional techniques for the prevention of fouling of the reactor and polymer agglomeration can be used in the practice of our invention. Illustrative of these techniques are the introduction of finely divided particulate matter to prevent agglomeration, as described in U.S. Patent Nos. 4,994,534 and 5,200,477; the addition of negative charge generating chemicals to balance positive voltages or the addition of positive charge generating chemicals to neutralize negative voltage potentials as described in U.S. Patent No. 4,803,251. Antistat substances may also be added, either continuously or intermittently to prevent or neutralize static charge generation.

The granular polybutadiene and/or polyisoprene elastomers of produced using the catalyst of the present invention can be compounded alone or in combination with other elastomers, e.g., natural rubber, styrene-butadiene rubber, (halo)butyl rubber, ethylene-propylene-diene rubber; reinforcing fillers, e.g. carbon black, silica; processing aids; antidegradants; and vulcanizing agents using equipment and methods well known to those skilled in the art. It is believed that in such compounds, the initially granular form of the. polybutadiene or polyisoprene permits more intimate mixing with the other elastomer(s), than would be. achievable with conventional polybutadiene or polyisoprene in solid bale form. It is generally desirable that elastomer blends be intimately mixed in order to optimize the mechanical properties of the vulcanizate. Furthermore, if the inert particulate material employed in this invention, used to maintain granularity during and after the polymerization process, also happens to be a reinforcing filler for the compound (e.g., carbon black), then a further benefit may be realized in the form of a shorter mixing time required to disperse the filler in the compound. This is because the filler, which normally would have to first be deagglomerated in the mixing process before it could be dispersed, in this case enters the mixing process already substantially deagglomerated and dispersed.

Elastomeric compounds prepared from granular polybutadiene and polyisoprene or mixtures thereof, are particularly useful as components of pneumatic tires. For example, as is known to those skilled in the tire making arts, in the production of a radial automobile tire, specially formulated elastomeric compounds can be extruded through a die to produce strip stock for the tread, sidewall, and bead filler components of the tire, or to produce sheet stock for the air retention innerliner. Other specially formulated elastomeric compounds can be calendered onto textile or steel cord fabric to produce cord-reinforced sheet stock for the carcass and circumferential belt components of the tire. The "green" or unvulcanized tire is built by assembling the various components (except circumferential belt and tread) on the surface of a cylindrical drum, radially expanding and axially compressing the assembly to produce a toroidal shape, then placing the belt and tread components in position around the circumference of the toroid. Finally, the.green tire is vulcanized by inflating with high pressure steam against the inner surface of a closed, heated aluminum mold. In the early stage of the vulcanization process, when the various elastomeric compounds are still soft and flowable, the pressure of the tire against the inner surface of the mold produces the final precise shape, tread pattern, sidewall lettering and decorative markings. Later in the vulcanization process, heat-activated crosslinking reactions take place within the various elastomeric compounds so that when the mold is finally opened each compound has undergone crosslinking to a degree that is essentially optimum for the intended purpose.

When used as a constituent of tire compounds, granular polybutadiene produced by using the catalyst of this invention particularly imparts abrasion resistance, fatigue cracking resistance, low heat generation, and low rolling resistance. Granular polyisoprene produced by using the catalyst of this invention particularly imparts building tack and green strength, which facilitate the building and handling of the green tire, and tear and cut resistance. The granular, free-flowing polybutadiene and polyisoprene produced using the catalyst of the present invention in a gas phase process can also be employed in other molded and extruded articles using techniques known to those skilled in the art.

The following examples are provided to illustrate our invention and its use in the polymerisation of olefins.

### Example 1

To a gas-phase stirred bed reactor that was maintained at a constant temperature of 60°C, 1.71 kg (3.8 pounds)of dried carbon black powder were added to act as a fluidization aid. To this was added 0.025 kg (0.055 lbs) TIBA, i.e. triisobutylaluminum. Then was added 0.84 kg (1.86 lbs) of 1,3-butadiene and sufficient nitrogen to bring the total reactor pressure to 2271 kPa (315 psia). A small feed of supported catalyst consisting of neodymium neodecanoate on DEAC-treated silica was begun. Simultaneously, a small feed of 10 wt% triisobutylaluminum co-catalyst solution in isopentane was begun. Feed was adjusted to give a 7:1 molar ratio of Al:Nd. During a 2.8 hour polymerization reaction, a total of 3.12 kg (6.93 lbs) of additional butadiene were fed in order to replace butadiene that was polymerized or vented. A small vent stream leaving the reactor removed a total of 0.43 kg (0.95 lbs) butadiene during the polymerization. At the end of the polymerization, the catalyst and co-catalyst feeds were stopped. The reactor was depressurized, and the reactor contents purged free of residual butadiene using nitrogen. The polymer was discharged from the reactor. The product did not contain any lumps that would indicate agglomeration had occurred. To the contrary, the product was a free-flowing, fine, granular powder. The reactor was opened and cleaned to ensure that all product was recovered. The total weight of solid product that was recovered was adjusted for the carbon black that had been initially charged. The remainder (2.40 kg (5.35 lbs) was the amount of butadiene polymer formed during the batch and which was present in the reactor when it was shut down. Since a total of 3.98 kg [8.79 lbs (= 6.93 + 1.86)] of butadiene were charged to the reactor and a total of 2.84 kg [6.30 lbs (= 5.35 + 0.95)] of butadiene have been accounted for leaving the reactor as polymer and in the continuous vent stream, there must have been 1.12 kg (2.49 lbs) of butadiene monomer present in the reactor when polymerization was terminated. This monomer would have been removed from the reactor when it was depressurized and the contents purged.

The reactor volume is 61.7 liters (or 2.18 cubic feet). At 60°C the vapor pressure of 1,3-butadiene is 810 kPa (103 psia). The mass of butadiene present in the reactor as a gas at saturation would thus be 0.85 kg (1.88 lbs). Of the total of 1.12 kg (2.49 lbs) of unpolymerized butadiene that was shown to be present in the reactor at shutdown, at most 0.85 kg (1.88 lbs) could have been in the vapor phase and the rest 0.27 kg (0.61 lbs) must have been present in a condensed phase, for example, dissolved in the polymer. Thus the reactor was being operated at a temperature below the condensation temperature of the monomer present. The 0.27 kg (0.61 lbs) of liquid monomer combined with the 2.40 kg (5.35 lbs) of polymer amounts to 5.13 kg (11.4 lbs) of condensed butadiene monomer per 45 kg (100 lbs) of polybutadiene. Yet, the presence of this liquid monomer in the gas-phase reactor did not cause agglomeration of the polymer.

Properties of the above product are as follows:
Carbon black N-650 by analysis: 41%;
Average particle size by sieve analysis; 0.64 mm (0.025 inches);
Neodymium in polymer: 490 ppm;
Reduced viscosity: 7.6 dl/g;
Cis 1-4: 97.6%

Examples 2-7 were conducted as in Example 1, but with the changes indicated in the tables.

Supported Catalyst Preparation for Example 2 To a 500 mL dry nitrogen purged flask was added 78.15 grams of silica (600°C activation) and 250 mL dry hexane. Slowly, 40 mL of 1.5M Et₂AlCl was added and the mixture was stirred for 60 minutes at room temperature. The solution was cooled and 117 grams of a hexane solution of neodymium versatate (4.9 wt% Nd) was added slowly. The mixture was stirred for 30 minutes and then the solvent was removed under vacuum.

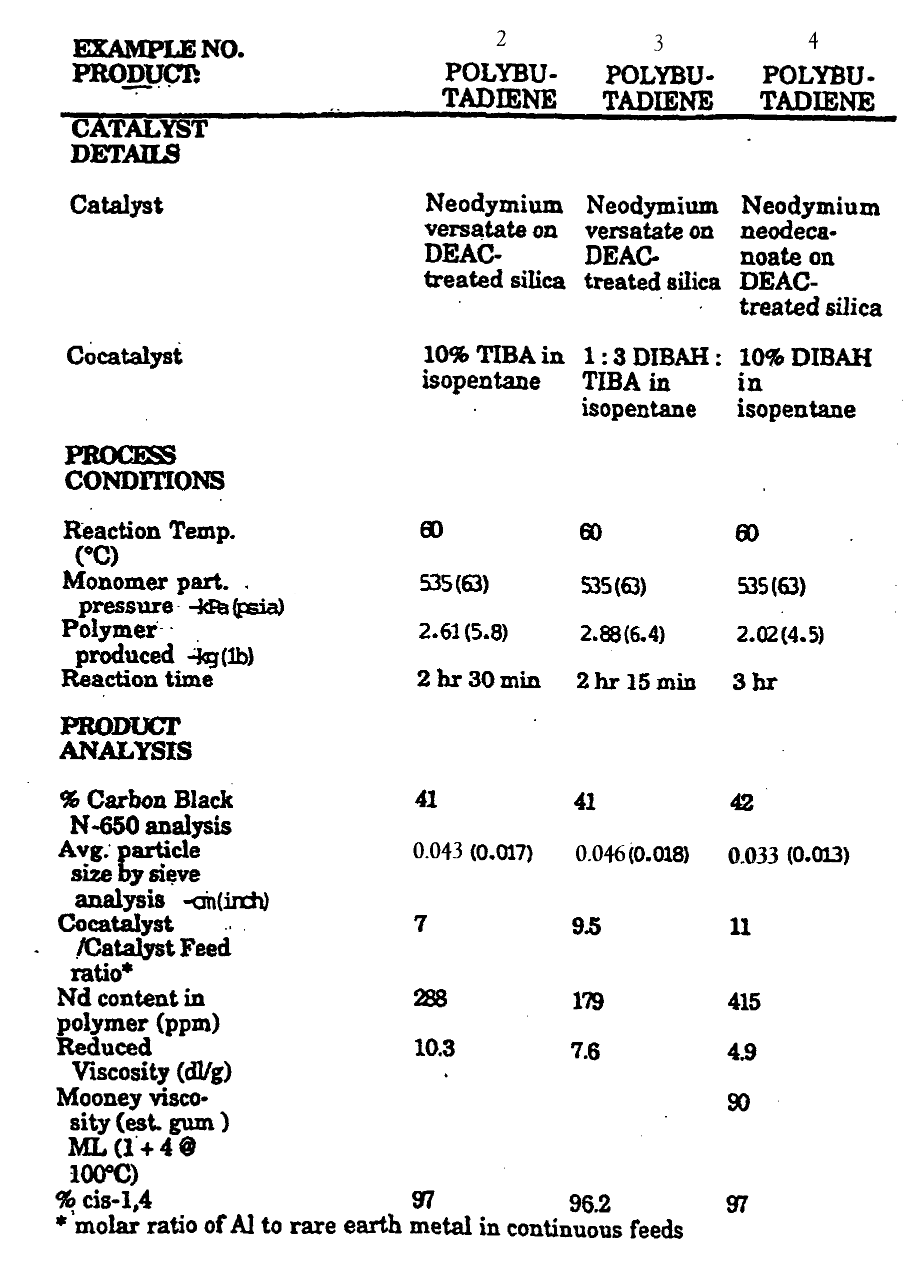

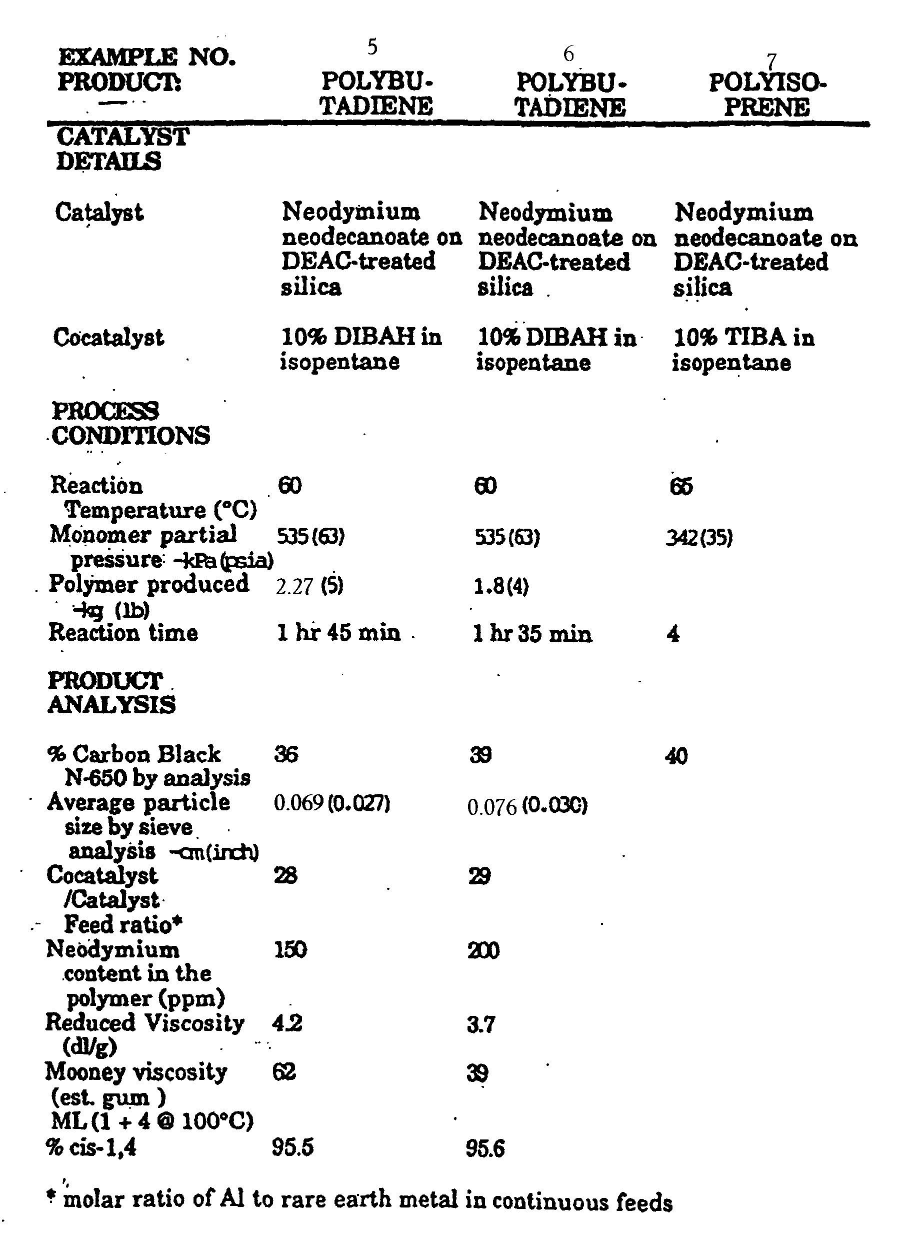

### Example 8

In an example of the catalyst of the present invention being used in a polymerisation reaction a fluidized bed reaction system as described above, is operated as described below to produce polybutadiene. The polymer is produced under the following reaction conditions: 60°C reactor temperature and 907 kPa (120 psia) total reactor pressure. The partial pressure of the butadiene monomer inside the reactor is 762 kPa (96 psia). The partial pressure of nitrogen is 264 kPa (24 psia). The catalyst system employed in this Example is neodymium neodecanoate supported on DEAC-treated silica with triisobutylaluminum as co-catalyst. Catalyst and co-catalyst feeds are adjusted to give a 60:1 molar ratio of Al to Nd. At steady state the monomer is fed into the reaction system at the rate of 20.7 kg/h (46.2 lb/h). Dried N-650 carbon black is fed to the reactor at the rate of 9 kg/h (20 lb/h). Butadiene monomer leaves the reactor at 5.85 kg/h (13 lb/h) in vent streams. The production rate is 13.6 kg/h ((3 lb/h) of polymer after adjusting for the carbon black content. The product has a Mooney viscosity ML (1 + 4 @ 100°C) of 55. Other conditions are shown for Example 9 in the table.

At steady state a total of 20.7 kg/h (46.2 lb/h) butadiene is being fed to the reactor and a total of 19.35 kg/h (43 lb/h) is accounted for leaving the reactor as gas in a vent stream or as polymer. The difference of 1.44 kg/h (3.2 lb/h) must be unreacted liquid butadiene monomer in the polymer leaving the reactor. Since the polymer discharged is identical with the polymer in the bed, the polymer in the bed must contain the same proportion of liquid monomer, i.e. there must be 5.35 kg (11.9 lbs) of dissolved liquid monomer in the 50.4 kg (112 lbs) polymer bed.

The reactor volume is 1.54 m³ (55 ft³). At the partial pressure of 762 kPa (96 psia), there are 19.98 kg (44.4 lbs) of butadiene in the reactor gas-phase. The total unpolymerized butadiene in the reactor is thus 25.3 kg [56.3 lbs (=44.4 + 11.9)]. If all of this butadiene were in the gas phase of this reactor at once it would have a partial pressure of 962 kPa (125 psia) and its condensation temperature would be 69°C. Therefore the reactor at 60°C is being operated below the condensation temperature of the monomer present in the polymerization zone. Furthermore, the presence of this liquid monomer in the gas-phase reactor does not cause agglomeration of the polymer.

### Example 9

In an example of the process of the invention a fluidized bed reaction system as described above, is operated as described below to produce polyisoprene. The polymer is produced under the following reaction conditions: 65°C reactor temperature and 100 psia total reactor pressure. The partial pressure of the isoprene monomer inside the reactor is 307 kPa (30 psia). The partial pressure of nitrogen is 583 kPa (70 psia). The catalyst system employed in this Example is neodymium neodecanoate supported on DEAC-treated silica with triisobutylaluminum as co-catalyst. Catalyst and co-catalyst feeds are adjusted to give a 60:1 molar ratio of Al to Nd. At steady state the monomer is fed into the reaction system at the rate of 15.9 kg/h (35.4 lb/h). Dried N-650 carbon black is fed to the reactor at the rate of 9 kg/h (20 lb/h). Isoprene monomer leaves the reactor at 0.9 kg/h (2 lb/h) in vent streams. The production rate is 13.5 kg/h (30 lb/h) of polymer after adjusting for the carbon black content. The product has a Mooney viscosity ML (1 + 4 @ 100°C) of 55. Other conditions are shown for Example 11 in the table.

At steady state a total of 15.93 kg/h (35.4 lb/h) isoprene is being fed to the reactor and a total of 14.4 kg/h (32 lb/h) is accounted for leaving the reactor as gas in a vent stream or as polymer. The difference of 1.53 kg/h (3.4 lb/h) must be unreacted liquid isoprene monomer in the polymer leaving the reactor. Since the polymer discharged is identical with the polymer in the bed, the polymer in the bed must contain the same proportion of liquid monomer, i.e. there must be 5.71 kg (12.7 lbs) of dissolved liquid monomer in the 50.4 kg (112 lbs) polymer bed.

The reactor volume is 1.54 m³ (55 ft³). At the partial pressure of 307 kPa (30 psia) there are 7.74 kg (17.2 lbs) of isoprene in the reactor gas-phase. The total unpolymerized isoprene in the reactor is thus 13.45 kg [29.9 lbs (=17.2 + 12.7)]. If all of this isoprene were in the gas phase of this reactor at once it would have a partial pressure of 416 kPa (54.5 psia) and its condensation temperature would be 80°C. Therefore the reactor at 65°C is being operated below the condensation temperature of the monomer present in the polymerization zone. Furthermore, the presence of this liquid monomer in the gas-phase reactor does not cause agglomeration of the polymer.

| **EXAMPLE NO. PRODUCT:** | **8 POLYBUTADIENE** | **9 POLYISOPRENE** |
|---|---|---|
| **REACTION CONDITIONS:** | | |
| Temperature (°C) | 60 | 65 |
| Total Pressure | 928 (120) | 790(100) |
| -kPa (psia) | | |
| Superficial Velocity m/s(ft/s) | 0.53(1.75) | 0.53(1.75) |
| Production Rate -kg/h (lb/h) | 13.5 (30) | 13.5(30) |
| Total Reactor | 1.54 (55) | 1.54(55) |
| Volume -m³(ft³) Reaction Zone | 0.21(7.5) | 0.21 (75) |
| Volume -m³(ft³) Bed Height -m(ft): | 2.1(7.0) | 2.1(7.0) |
| Bed Diameter -m(ft) | 0.35 (1.17) | 0.35(1.17) |
| Bed Weight kg(lbs) | 50.4(112) | 50.4(112) |

| **CYCLE GAS COMPOSITION (MOLE %):** | | |
|---|---|---|
| N₂ | 20 | 70 |
| Butadiene | 80 | - |
| Isoprene | - | 30 |

| CATALYST: | Nd Neodecanoate on DEAC-treated silica. | Nd Neodecanoate on DEAC-treated silica |
|---|---|---|
| **CO-CATALYST:** | **TIBA** | **TIBA** |
| Monomer Feed | | |
| Rate -kg/h(lb/h) | | |
| Butadiene | 13.86 (46.2) | -- |
| Isoprene | - | 10.6(35.4) |
| Monomer Vent | 3.9 (13) | 0.6(2) |
| Rate -kg/h(lb/hr) | | |

| **POLYMER COMPOSITION (wt%):** | | |
|---|---|---|
| Butadiene | 100 | -- |
| Isoprene | -- | 100 |

## Claims

1. A polymerisation catalyst comprising:
(A) rare earth metal component selected from the group consisting of (1) a reaction mixture obtainable by treating a silica support, a carbon black support or a mixed support thereof with a mono- or di-ethyl aluminium chloride, adding a neodymium carboxylate in the presence of a solvent to form a slurry, and removing the solvent or (2) a reaction mixture obtainable by dissolving a neodymium carboxylate in a first solvent, adding silica or carbon black, and removing the solvent to obtain a dry solid, contacting the dry solid with a solution containing a mono- or di-ethyl aluminium chloride in a second solvent, and removing the second solvent; and
(B) a co-catalyst selected from the group consisting of a diisobutyl aluminium hydride, triisobutyl aluminium, and a mixture thereof.

2. A polymerisation catalyst as claimed in claim 1 wherein the rare earth component (A) is obtainable by the sequential steps of:
(i) treating a silica support, carbon black support, or mixed support thereof with diethyl aluminium chloride in a solvent to form a slurry;
(ii) adding a neodymiuim carboxylate to the slurry; and
(iii) removing the solvent.

3. A polymerisation catalyst as claimed in claim 1 wherein the rare earth component (A) is obtainable by the sequential steps of
(i) impregnating a silica support or a carbon black support with a neodymium carboxylate in a first solvent to form a slurry;
(ii) removing the first solvent from step (i) to obtain a dry solid;
(iii) contacting said dry solid with diethyl aluminium chloride in a second solvent to form a slurry; and
(iv) removing the second solvent from step (iii), wherein the first solvent of step (i) and the second solvent of step (iii) may be the same or different.

4. A polymerisation catalyst as claimed in any one of the preceding claims wherein the neodymium carboxylate is neodymium neodecanoate, neodymium octoate or neodymium versatate.

5. A polymerisation catalyst as claimed in any one of the preceding claims wherein the organic alkyl aluminium compound is diethyl aluminium chloride.

6. A polymerisation catalyst as claimed in claim 2 wherein the solvent is n-hexane or cyclohexane.

7. A polymerisation catalyst as claimed in claim 3 wherein the first solvent is n-hexane or cyclohexane and the second solvent is a hydrocarbon.

## Patentansprüche

1. Polymerisationskatalysator umfassend:
(A) eine Seltenerdmetall-Komponente ausgewählt aus der Gruppe bestehend aus (1) einer Reaktionsmischung erhältlich durch Behandeln eines Siliciumdioxid-Trägers, eines Ruß-Trägers oder eines gemischten Trägers davon mit einem Mono- oder Diethylaluminiumchlorid, Zugabe eines Neodymcarboxylats in Anwesenheit eines Lösungsmittels, um eine Aufschlämmung zu bilden, und Entfernen des Lösungsmittels oder (2) einer Reaktionsmischung erhältlich durch Lösen eines Neodymcarboxylats in einem ersten Lösungsmittel, Zugabe von Siliciumdioxid oder Ruß und Entfernen des Lösungsmittels, um einen trockenen Feststoff zu erhalten, Kontaktieren des trockenen Feststoffs mit einer Lösung, die ein Mono- oder Diethylaluminiumchlorid in einem zweiten Lösungsmittel enthält, und Entfemen des zweiten Lösungsmittels, und
(B) einen Cokatalysator ausgewählt aus der Gruppe bestehend aus Diisobutylaluminiumhydrid, Triisobutylaluminium und einer Mischung davon.

2. Polymerisationskatalysator wie in Anspruch 1 beansprucht, worin die Seltenerd-Komponente (A) erhältlich ist durch die aufeinanderfolgenden Schritte
(i) des Behandelns eines Siliciumdioxid-Trägers, eines Ruß-Trägers oder eines gemischten Trägers davon mit Diethylaluminiumchlorid in einem Lösungsmittel, um eine Aufschlämmung zu bilden,
(ii) der Zugabe eines Neodymcarboxylats zur Aufschlämmung und
(iii) des Entfernens des Lösungsmittels.

3. Polymerisationskatalysator wie in Anspruch 1 beansprucht, worin die Seltenerd-Komponente (A) erhältlich ist durch die aufeinanderfolgenden Schritte
(i) des Imprägnierens eines Siliciumdioxid-Trägers oder eines Ruß-Trägers mit einem Neodymcarboxylat in einem ersten Lösungsmittel, um eine Aufschlämmung zu bilden,
(ii) des Entfernens des ersten Lösungsmittels von Schritt (i), um einen trockenen Feststoff zu erhalten,
(iii) des Kontaktierens des trockenen Feststoffs mit Diethylaluminiumchlorid in einem zweiten Lösungsmittel, um eine Aufschlämmung zu bilden, und
(iv) des Entfernens des zweiten Lösungsmittels von Schritt (iii), wobei das erste Lösungsmittel von Schritt (i) und das zweite Lösungsmittel von Schritt (iii) gleich oder verschieden sein können.

4. Polymerisationskatalysator wie in irgendeinem der vorhergehenden Ansprüche beansprucht, worin das Neodymcarboxylat Neodymneodecanoat, Neodymoctoat oder Neodymversatat ist.

5. Polymerisationskatalysator wie in irgendeinem der vorhergehenden Ansprüche beansprucht, worin die organische Alkylaluminiumverbindung Diethylaluminiumchlorid ist.

6. Polymerisationskatalysator wie in Anspruch 2 beansprucht, worin das Lösungsmittel n-Hexan oder Cyclohexan ist

7. Polymerisationskatalysator wie in Anspruch 3 beansprucht, worin das erste Lösungsmittel n-Hexan oder Cyclohexan ist und das zweite Lösungsmittel ein Kohlenwasserstoff ist.

## Revendications

1. Catalyseur de polymérisation qui comprend :
(A) un constituant renfermant un métal des terres rares, qui est choisi parmi (1) un mélange réactionnel que l'on peut obtenir en traitant un support de silice, un support de noir de carbone ou un support mixte de silice et de noir de carbone, avec un chlorure de mono- ou diéthylaluminium, en ajoutant un carboxylate de néodyme en présence d'un solvant pour former une bouillie, et en éliminant le solvant, et (2) un mélange réactionnel que l'on peut obtenir en dissolvant un carboxylate de néodyme dans un premier solvant, en ajoutant de la silice ou du noir de carbone, en éliminant le solvant pour obtenir un solide sec, en mettant le solide sec en contact avec une solution contenant du chlorure de mono- ou diéthylaluminium dans un second solvant, et en éliminant le second solvant, et
(B) un cocatalyseur choisi parmi l'hydrure de diisobutylaluminium, le triisobutylaluminium et leurs mélanges.

2. Catalyseur de polymérisation selon la revendication 1, dont le constituant (A) renfermant un métal des terres rares peut être obtenu par les étapes successives suivantes :
(i) le traitement d'un support de silice, d'un support de noir de carbone ou d'un support mixte de silice et de noir de carbone avec du chlorure de diéthylaluminium dans un solvant pour former une bouillie,
(ii) l'addition d'un carboxylate de néodyme à la bouillie, et
(iii) l'élimination du solvant.

3. Catalyseur de polymérisation selon la revendication 1, dont le constituant (A) renfermant un métal des terres rares peut être obtenu par les étapes successives suivantes :
(i) l'imprégnation d'un support de silice ou d'un support de noir de carbone avec un carboxylate de néodyme dans un premier solvant pour former une bouillie,
(ii) l'élimination du premier solvant de la bouillie de l'étape (i) pour obtenir un solide sec,
(iii) la mise en contact dudit solide sec avec du chlorure de diéthylaluminium dans un second solvant pour former une bouillie, et
(iv) l'élimination du second solvant de la bouillie de l'étape (iii), le premier solvant de l'étape (i) et le second solvant de l'étape (iii) pouvant être identiques ou différents.

4. Catalyseur de polymérisation selon l'une quelconque des revendications précédentes, pour lequel le carboxylate de néodyme est le néodécanoate de néodyme, l'octoate de néodyme ou le versatate de néodyme.

5. Catalyseur de polymérisation selon l'une quelconque des revendications précédentes, pour lequel le composé organique alkylaluminium est le chlorure de diéthylaluminium.

6. Catalyseur de polymérisation selon la revendication 2, pour lequel le solvant est le n-hexane ou le cyclohexane.

7. Catalyseur de polymérisation selon la revendication 3, pour lequel le premier solvant est le n-hexane ou le cyclohexane et le second solvant est un hydrocarbure.
